# FASCICULE DE BREVET EUROPEEN

(11) **EP 0 379 416 B1**
(45) Date de publication et mention de la délivrance du brevet: **08.03.1995**
(21) Numéro de dépôt: 90400110.4
(22) Date de dépôt: 16.01.1990
(51) Int. Cl.: C07D 498/04, C07D 487/04, C07D 471/04, A61K 31/41, A61K 31/435, A61K 31/55

(54) **Dérivés d'azabicycloheptène et leurs sels, leur procédé de préparation, leur application comme médicaments et les compositions les renfermant**
Azabicycloheptenderivate und ihre Salze, Verfahren zu ihrer Herstellung, ihre Verwendung als Arzneimittel und diese enthaltende Zubereitungen
Azabicycloheptene derivatives and their salts, process for their preparation, their use as medicaments and compositions containing them

(30) Priorité: 16.01.1989 GB 8900863
(43) Date de publication de la demande: 25.07.1990
(73) Titulaire: ROUSSEL-UCLAF, 75007 Paris (FR)
(72) Inventeur: Luheshi, Abdul Basset Nuri, Hull (GB); Kennewell, Peter David, Swindon, Wiltshire (GB); Samlley, Robert Kenneth, Urmston, Manchester (GB); Westwood, Robert, Kingston Bagpuize, Oxon (GB)
(74) Mandataire: Jacobi, Markus Alexander

(56) Documents cités:
- DE-A- 2 155 753
- TETRAHEDRON LETTERS, vol. 31, no. 1, janvier 1990, pages 123-126, Oxford GB; R.K. SMALLEY et al.: "1,3-Dipolar cycloadditions of 2-ethoxy- and 2-(ethylthio)-1-azetines with nitrile oxides and nitrile ylides"
- TETRAHEDRON LETTERS, vol. 31, no. 1, janvier 1990, pages 127-130, Oxford, GB; R.K. SMALLEY et al.: "1,3-Dipolar cycloadditions of 2-ethoxy- and 2-(ethylthio)-1-azetines with nitrilimines"

## Description

La présente invention concerne de nouveaux dérivés d'azabicycloheptène et leurs sels ainsi que le procédé de préparation, leur application à titre de médicaments et les compositions les renfermant.

L'invention a pour objet de nouveaux dérivés d'azabicycloheptène répondant à la formule générale (I) : 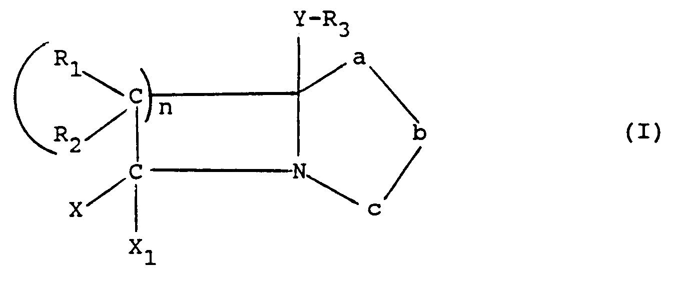
dans laquelle :
- R₁, R₂ identiques ou différents, représentent un atome d'hydrogène, un radical alkyle renfermant de 1 à 5 atomes de carbone,
- n représente un nombre entier égal à 1 ou 2,
- X et X₁ représentent chacun un atome d'hydrogène ou un radical alkyle renfermant de 1 à 3 atomes de carbone, ou X et X₁ forment ensemble avec l'atome de carbone auxquels ils sont liés un groupement O=C-,
- Y représente un atome d'oxygène ou un atome de soufre,
- R₃ représente un radical alkyle renfermant de 1 à 3 atomes de carbone ou un groupement phényle 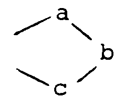 représente des groupements 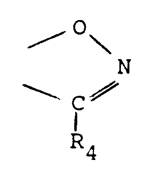 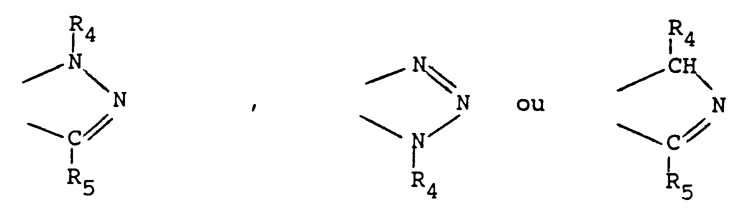 dans lesquels R₄ et R₅ identiques ou différents représentent un groupement phényle ou un groupement phényle substitué par un atome d'halogène, un radical alkyle renfermant de 1 à 3 atomes de carbone, un radical alcoxy renfermant de 1 à 3 atomes de carbone, un groupement nitro, ainsi que leurs sels.

Dans la formule générale (I) et dans ce qui suit,
- le terme radical alkyle renfermant de 1 à 5 atomes de carbone désigne par exemple un radical méthyle, éthyle, propyle, isopropyle ou butyle,
- le terme radical hydroxyalkyle renfermant de 1 à 5 atomes de carbone désigne par exemple un radical hydroxyméthyle, hydroxyéthyle, hydroxypropyle, hydroxybutyle,
- le terme radical alkyle renfermant de 1 à 3 atomes de carbone désigne par exemple un radical méthyle, éthyle, propyle ou isopropyle,
- le terme atome d'halogène désigne par exemple un atome de fluor, de chlore ou de brome,
- le terme radical alcoxy renfermant de 1 à 3 atomes de carbone désigne par exemple un radical méthoxy, éthoxy, propoxy ou isopropoxy.

Certains produits de formule (I) présentent un caractère basique ; ils peuvent par conséquent former des sels d'addition avec les acides tels que par exemple les sels formés avec acides chlorhydrique, bromhydrique, iodhydrique, nitrique, sulfurique, phosphorique, acétique, formique, benzoïque, maléique, fumarique, succinique, tartrique, citrique, oxalique, glyoxylique, aspartique alcanesulfoniques, tels que l'acide méthanesulfonique et arylsulfoniques, tels que l'acide benzènesulfonique.

Certains produits de formule (I) présentent un caractère acide ; ils peuvent par conséquent former des sels de métaux alcalins, alcalino-terreux ou avec les bases azotées.

Parmi les produits objet de l'invention, on peut citer notamment les dérivés répondant à la formule (I) ci-dessus dans laquelle :
- R₁, R₂, identiques ou différents, représentent un atome d'hydrogène, un radical méthyle, un radical méthoxy ou un radical amino,
- X et X₁, identiques ou différents, représentent un atome d'hydrogène ou un radical méthyle, ou X et X₁ forment ensemble avec l'atome de carbone auxquels ils sont liés un groupement 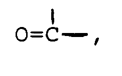
- R₃ représente un radical méthyle ou éthyle, n, Y et 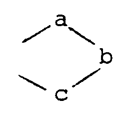 ont la signification déjà indiquée, ainsi que leurs sels.

On peut citer particulièrement les produits de formule (I) dans laquelle :
- R₁, R₂, X, X₁ représentent un radical méthyle,
- R₃ représente un radical éthyle,
- R₄ et R₅, identiques ou différents, représentent un groupement phényle ou un groupement phényle substitué par un radical méthoxy ou par un groupement nitro,
- n et Y ont la signification déjà indiquée, ainsi que leurs sels.

Parmi ces derniers, on peut citer plus particulièrement les dérivés répondant à la formule (I) ci-dessus dans laquelle n est égal à 1, ainsi que leurs sels et les produits de formule (I) ci-dessus dans laquelle n est égal à 2, ainsi que leurs sels.

Parmi les produits préférés de l'invention, on retient tout particulièrement les produits dont les noms suivent :
- le 3-(p-méthoxyphényl) 5,5,6,6-tétraméthyl 7-éthoxy 1-oxa 2,4-diazabicyclo [3.2.0.] hept-2-ène;
- le 3-phényl 5,5,6,6-tétraméthyl 7-éthoxy 1-oxa 2,4-diazabicyclo [3.2.0.] hept-2-ène;
- le 3-(O-nitrophényl) 5,5,6,6-tétraméthyl 7-éthoxy 1-oxa 2,4-diazabicyclo [3.2.0.] hept-2-ène;
- le 3-(p-méthoxyphényl) 5,5,6,6-tétraméthyl 7-éthylthio 1-oxa 2,4-diazabicyclo [3.2.0.] hept-2-ène;
- le 3-phényl 5,5,6,6-tétraméthyl 7-éthylthio 1-oxa 2,4-diazabicyclo [3.2.0.] hept-2-ène;
- le 3-(O-nitrophényl) 5,5,6,6-tétraméthyl 7-éthylthio 1-oxa 2,4-diazabicyclo [3.2.0.] hept-2-ène;
- le 7-éthoxy 1-(p-nitrophényl) 3-phényl 5,5,6,6-tétraméthyl 1,2,4-triazabicyclo [3.2.0.] hept-2-ène;
- le 7-éthylthio 1-(p-nitrophényl) 3-phényl 5,5,6,6-tétraméthyl 1,2,4-triazabicyclo [3.2.0.] hept-2-ène;
- le 7-éthoxy 3-phényl 1-(p-nitrophényl) 5,5,6,6-tétraméthyl 2,4-diazabicyclo [3.2.0.] hept-2-ène;
- le 7-éthylthio 3-phényl 1-(p-nitrophényl) 5,5,6,6-tétraméthyl 2,4-diazabicyclo [3.2.0.] hept-2-ène.

L'invention a également pour objet un procédé de préparation des nouveaux dérivés d'azabicycloheptène tels que définis par la formule (I) ci-dessus, ainsi que de leurs sels, caractérisé en ce que l'on effectue une cycloaddition entre un produit de formule (II) : 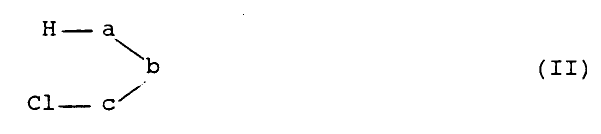
dans laquelle le groupement 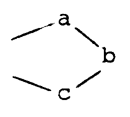
a la signification déjà indiquée et un produit de formule (III) : 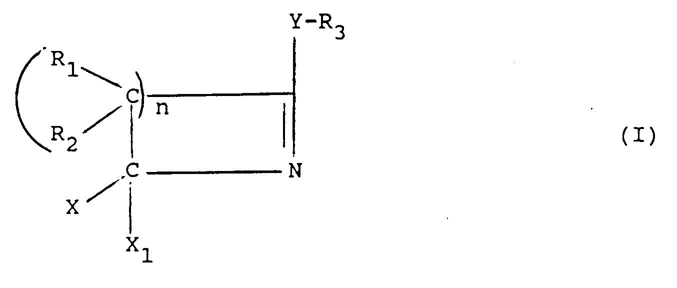
dans laquelle R₁, R₂, n, X, X₁, Y et R₃ ont la signification déjà indiquée, pour obtenir le produit de formule (I) dans laquelle R₁, R₂, n, X, X₁, Y, R₃, 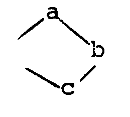
ont la signification déjà indiquée que l'on peut salifier le cas échéant.

Dans des conditions préférentielles de mise en oeuvre de l'invention, le procédé de préparation ci-dessus décrit est caractérisé en ce que la cycloaddition entre le produit de formule (II) et le produit de formule (III) est effectuée au sein d'un solvant organique apolaire tel que le benzène en présence d'une amine telle que la triéthylamine.

Lorsque l'on désire préparer un produit de formule (I) dans laquelle 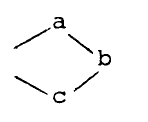
représente un groupement 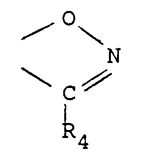
le produit de formule (II) est un produit de formule : 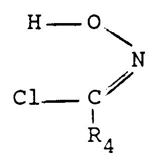
Lorsque l'on désire préparer un produit de formule (I) dans lequel 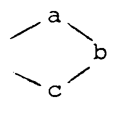
représente un groupement 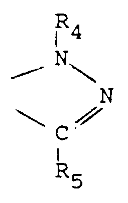
le produit de formule (II) est un produit de formule : 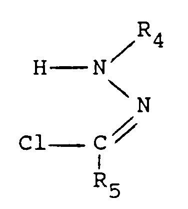
Lorsque l'on désire préparer un produit de formule (I) dans lequel 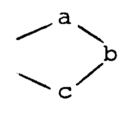
représente un groupement 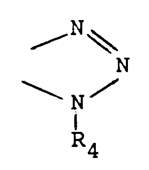
le produit de formule (II) est un produit de formule : 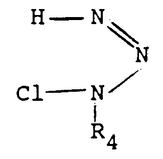
Lorsque l'on désire préparer un produit de formule (I) dans lequel 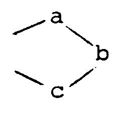
représente un groupement 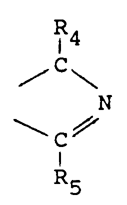
le produit de formule (II) est un produit de formule : 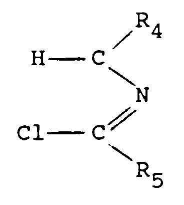
On peut avantageusement préparer les sels d'addition des produits de formule (I) en faisant réagir, en proportions sensiblement stoechiométriques, un acide minéral ou organique ou une base appropriée avec lesdits produits de formule (I). Les sels peuvent être préparés sans isoler les produits de formule (I) correspondants.

Les produits objet de la présente invention possèdent de très intéressantes propriétés pharmacologiques ; ils sont doués notamment de remarquables propriétés inhibitrices de la cyclo oxygénase (TX B₂ et 5-HHT). Ils possèdent également une activité antibiotique.

Ces propriétés sont illustrées plus loin dans la partie expérimentale.

Ces propriétés justifient l'utilisation des nouveaux dérivés d'azabicycloheptène objet de la présente demande, ainsi que de leurs sels pharmaceutiquement acceptables, à titre de médicaments.

La présente demande a ainsi, également pour objet, l'application à titre de médicaments, des nouveaux dérivés d'azabicycloheptène tels que définis par la formule (I) ci-dessus, ainsi que de leurs sels pharmaceutiquement acceptables.

Parmi les médicaments objet de l'invention, on retient de préférence les médicaments caractérisés en ce qu'ils sont constitués par les dérivés répondant à la formule (I) ci-dessus, dans laquelle :
- R₁, R₂, identiques ou différents, représentent un atome d'hydrogène, un radical méthyle, un radical méthoxy ou un radical amino,
- X et X₁, identiques ou différents, représentent un atome d'hydrogène ou un radical méthyle, ou X et X₁ forment ensemble avec l'atome de carbone auxquels ils sont liés un groupement 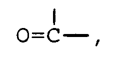
- R₃ représente un radical méthyle ou éthyle, n, Y et 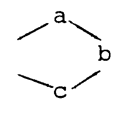 ont la signification déjà indiquée, ainsi que leurs sels pharmaceutiquement acceptables..

Parmi ceux-ci, on retient notamment les médicaments, caractérisés en ce qu'ils constitués par les dérivés répondant à la formule (I) dans laquelle :
- R₁, R₂, X, X₁ représentent un radical méthyle,
- R₃ représente un radical éthyle,
- R₄ et R₅, identiques ou différents, représentent un groupement phényle ou un groupement phényle substitué par un radical méthoxy ou par un groupement nitro,
- n et Y ont la signification déjà indiquée, ainsi que leurs sels pharmaceutiquement acceptables.

Parmi les médicaments de l'invention, on retient plus particulièrement ceux pour lesquels n est égal à 1 et ceux pour lesquels n est égal à 2, ainsi que leurs sels d'addition avec les acides pharmaceutiquement acceptables.

Enfin, parmi les médicaments préférés de l'invention, on retient tout particulièrement les produits dont les noms suivent :
- le 3-(p-méthoxyphényl) 5,5,6,6-tétraméthyl 7-éthoxy 1-oxa 2,4-diazabicyclo [3.2.0.] hept-2-ène;
- le 3-phényl 5,5,6,6-tétraméthyl 7-éthoxy 1-oxa 2,4-diazabicyclo [3.2.0.] hept-2-ene;
- le 3-(O-nitrophényl) 5,5,6,6-tétraméthyl 7-éthoxy 1-oxa 2,4-diazabicyclo [3.2.0.] hept-2-ène;
- le 3-(p-méthoxyphényl) 5,5,6,6-tétraméthyl 7-éthylthio 1-oxa 2,4-diazabicyclo [3.2.0.] hept-2-ène;
- le 3-phényl 5,5,6,6-tétraméthyl 7-éthylthio 1-oxa 2,4-diazabicyclo [3.2.0.] hept-2-ène;
- le 3-(O-nitrophényl) 5,5,6,6-tétraméthyl 7-éthylthio 1-oxa 2,4-diazabicyclo [3.2.0.] hept-2-ène;
- le 7-éthoxy 1-(p-nitrophényl) 3-phényl 5,5,6,6-tétraméthyl 1,2,4-triazabicyclo [3.2.0.] hept-2-ène;
- le 7-éthylthio 1-(p-nitrophényl) 3-phényl 5,5,6,6-tétraméthyl 1,2,4-triazabicyclo [3.2.0.] hept-2-ène;
- le 7-éthoxy 3-phényl 1-(p-nitrophényl) 5,5,6,6-tétraméthyl 2,4-diazabicyclo [3.2.0.] hept-2-ène;
- le 7-éthylthio 3-phényl 1-(p-nitrophényl) 5,5,6,6-tétraméthyl 2,4-diazabicyclo [3.2.0.] hept-2-ène,
ainsi que leurs sels pharmaceutiquement acceptables.

Ces médicaments trouvent par exemple leur emploi dans le traitement des maladies inflammatoires, des maladies du système immuno-régulateur, des syndromes dans lesquels les leucotriènes sont en jeu, ainsi que dans le traitement des affections provoquées par des bactéries à gram (+) et à gram (-).

La dose usuelle variables selon le produit utilisé, le sujet traité et l'affection en cause peut être, par exemple, de 0,25 mg à 100 mg par jour, par voie orale chez l'homme.

L'invention a enfin pour objet les compositions pharmaceutiques qui renferment au moins un dérivé précité ou l'un de ses sels pharmaceutiquement acceptables, à titre de principe actif.

A titre de médicaments, les dérivés répondant à la formule (I) et leurs sels pharmaceutiquement acceptables peuvent être incorporés dans des compositions pharmaceutiques destinées à la voie digestive ou parentérale.

Ces compositions pharmaceutiques peuvent être par exemple solides ou liquides et se présenter sous les formes pharmaceutiques couramment utilisées en médecine humaine, comme par exemple, les comprimés, simples ou dragéifiés, les gélules, les granulés, les suppositoires, les sirops, les aérosols, les préparations injectables ; elles sont préparées selon les méthodes useulles. Le ou les principes actifs peuvent y être incorporés à des excipients habituellement employés dans ces compositions pharmaceutiques, tels que talc, la gomme arabique, la lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Certains produits de formule (III) peuvent être préparés par un procédé analogue à celui décrit dans la demande de brevet allemand OLS 1770556.

Lorsqu'ils ne sont pas connus, les produits de formule (III) peuvent être préparés en utilisant le schéma suivant : 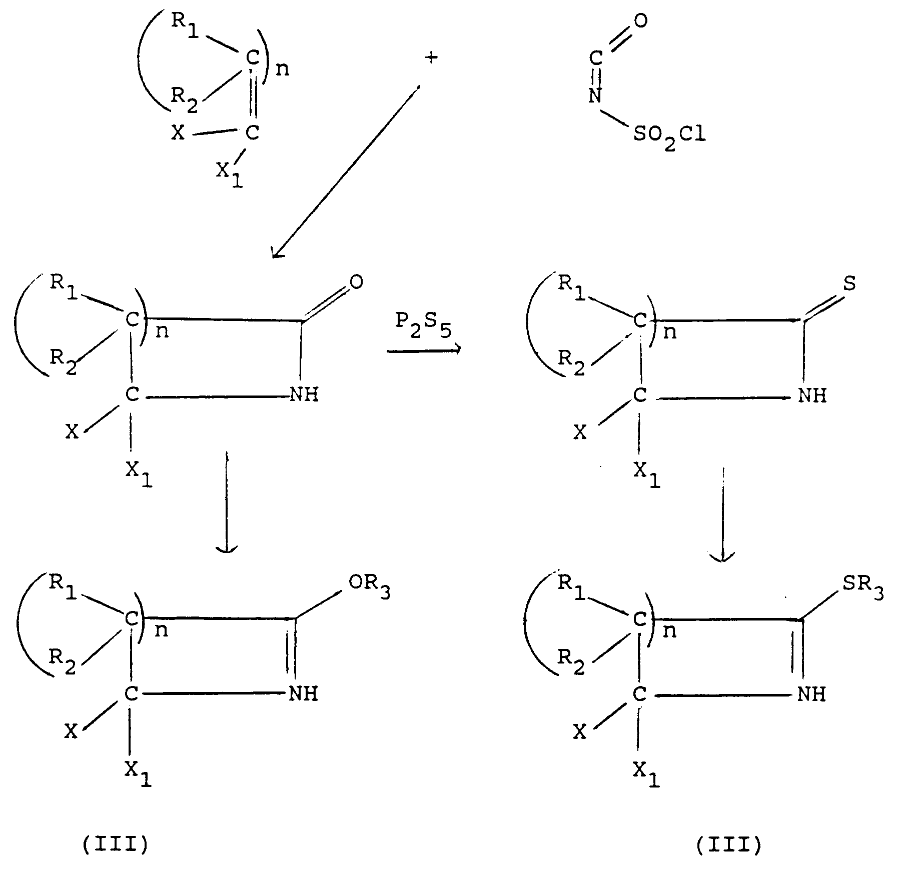
Des exemples d'une telle préparation fgurent plus loin dans la partie expérimentale.

Les produits de formule (II) peuvent être préparés selon des méthodes connues comme par exemple celle décrite dans R. Huisgen - Angewandte Chem. (1963) 2, 565.

Il va être donné maintenant à titre non limitatif des exemples de mise en oeuvre de l'invention.

### Exemple 1 : 3-(p-méthoxyphényl) 5,5,6,6-tétraméthyl 7-éthoxy 1-oxa 2,4-diazabicyclo [3.2.0.] hept-2-ène.

On ajoute sous agitation, 0,17 cm₃ de triéthylamine dans une solution comprenant 200 mg de 2-éthoxy 3,3,4,4-tétraméthyl 1-azétine et 240 mg de chlorure d'hydroxymoyle dans 20 cm₃ de benzène. On agite 2 heures à température ambiante, élimine par filtration le chlorhydrate de triéthylammonium et le lave au chlorure de méthylène, chromatographie le résidu et obtient le produit attendu pur. F = 107-109°C.
Spectre RMN :
epsilon 0,96(s,3H); epsilon 1,19(m,9H); epsilon 1,35(s,3H); epsilon 3,22(dq,2H); epsilon 3,88(s,3H); epsilon 6,92(d,2H); epsilon 7,53(d,2H).

| Analyse : C₁₇H₂₄N₂O₃ | | | | | | |
|---|---|---|---|---|---|---|
| Calculé | C% | 67,08 | H% | 7,95 | N% | 9,20 |
| Trouvé | | 66,80 | | 7,93 | | 9,12 |

### Préparation du 2-éthoxy 3,3,4,4-tétraméthyl 1-azétine utilisé au départ de l'exemple 1.

### Stade A : 1-chlorosulfonyl 3,3,4,4-tétraméthyl azétidinone.

On ajoute sous atmosphère inerte à 30-35°C d'isocyanate de chlorosyulfonyle en soyution dans l'éther à 2,3-diméthyl but-2-ène. On agite 1 heure, laisse revenir à température ambiante puis glace. On filtre le produit cristallisé que l'on utilise tel quel pour le stade suivant.

### Stade B : 3,3,4,4-tétraméthyl 1-azétidine 2-one.

On ajoute lentement sous agitation 10 g de produit obtenu au stade A en solution dans l'éther à un mélange comprenant 20 cm₃ d'eau, 25 g de glace, 12 g de bicarbonate de sodium et 8 g de sulfite de sodium heptahydraté en maintenant la température entre 0 et 5°C. On agite 1 heure, filtre, sépare la phase organique et extrait la phase aqueuse au chlorure de méthylène. On réunit les phases organiques, les sèche et élimine les solvants sous pression réduite. On recueille le produit attendu.
Spectre RMN :
1,18 (s,6H,2Me) ; 1,26(s,6H,2Me) ; 6,95(s,1H,NH).

### Stade C : 2-éthoxy 3,3,4,4-tétraméthyl 1-azétine.

A 60 cm₃ d'une solution 1M de tétrafluoroborate de triéthyloxonium dans le dichlorométhane, on ajoute sous atmosphère inerte 5 g de produit obtenu au stade B dans 10 cm₃ de chlorure de méthylène. On agite 1 heure à température ambiante puis chauffe 1 heure au reflux. Après refroidissement, on verse le milieu réactionnel dans 20 cm₃ d'une solution de carbonate de potassium à 50% refroidie à -10°C. On dilue le mélange avec 20 cm₃ de chlorure de méthylène et filtre. On sépare la phase organique, la sèche et élimine le solvant sous pression réduite. On distille le résidu à 78-80°C sous 50 mm de mercure et obtient le produit attendu.
Spectre RMN :
Epsilon 1,12(s,6H) ; 1,18(s,6H) ; 1,26(t,3H) ; 4,12(q,2H).
Spectre IR :
C=N : 1630 cm⁻¹
En opérant comme à l'exemple 1, en utilisant au départ les produits appropriés, on a préparé les composés suivants :

### Exemple 2 : 3-phényl 5,5,6,6-tétraméthyl 7-éthoxy 1-oxa 2,4-diazabicyclo [3.2.0.] hept-2-ène.

F = 106-108°C.

### Exemple 3 : 3-(O-nitrophényl) 5,5,6,6-tétraméthyl 7-éthoxy 1-oxa 2,4-diazabicyclo [3.2.0.] hept-2-ène.

F = 103-104°C.

### Exemple 4 : 3-(p-méthoxyphényl) 5,5,6,6-tétraméthyl 7-éthylthio 1-oxa 2,4-diazabicyclo [3.2.0.] hept-2-ène.

On ajoute sous atmosphère inerte 0,17 cm₃ de triéthylamine à une solution comprenant 200 mg de 2-éthylthio 3,3,4,4-tétraméthyl 1-azétine et 220 mg de chlorure d'hydroxymoyle dans 20 cm₃ de benzène. On agite 2 heures à température ambiante, filtre le chlorhydrate de triéthylammonium, le lave au chlorure de méthylène. On réunit les phases organiques, élimine les solvants sous pression réduite, chromatographie le résidu et obtient le produit attendu. F = 106-108°C.
Spectre RMN :
Epsilon 1,0(s,3H) ; 1,12(m,9H) ; 1,44(s,3H) ; 2,55 (dq,2H) ; 3,68(s,3H) ; 6,93(d,2H) ; 7,53 (d,2H).

| Analyse : C₁₇H₂₄N₂O₂S | | | | | | | | |
|---|---|---|---|---|---|---|---|---|
| Calculé | C% | 63,75 | H% | 7,50 | N% | 8,75 | S% | 10,00 |
| Trouvé | | 63,56 | | 7,52 | | 8,69 | | 9,94 |

### Préparation du 2-éthylthio 3,3,4,4-tétraméthyl 1-azétine utilisé au départ de l'exemple 4.

### a) 3,3,4,4-tétraméthyl azétirinthione.

On ajoute sous atmosphère inerte 1,59 g de réactif de Lawesson à 1 g de 2-éthoxy 3,3,4,4-tétraméthyl 1-azétine en solution dans 15 cm₃ de tétrahydrofuranne et agite 20 minutes. On chauffe à 60°C pendant 20 minutes, refroidit, élimine le solvant sous pression réduite, chromatographie le résidu sur silice (éluant : éther de pétrole-acétate d'éthyle 7-3) et récupère le produit attendu. F = 117-118°C.
Spectre RMN :
1,2(s,6H,2Me) ; 1,32(s,6H,2Me) ; 8,3(large s,1H,NH).

### b) 2-éthylthio 3,3,4,4-tétraméthyl-1-azétine.

On ajoute sous atmosphèe inerte 1 g de produit préparé au stade a) en solution dans 3 cm₃ de chlorure de méthylène dans 10,5 cm₃ d'une solution 1M de tétrafluoroborate de triéthylamine dans le chlorure de méthylène. On agite 1 heure à température ambiante, chauffe 1 heure au reflux, refroidit le milieu réactionnel, le verse dans 10 cm₃ d'une solution aqueuse refroidie à -10°C de bicarbonate de potassium à 50%, filtre, sépare la phase organique, la sèche, élimine le solvant sous pression réduite, chromatographie le résidu sur silice (éluant : éther de pétrole-acétate d'éthyle 8-2) et récupère le produit attendu.
Spectre RMN :
Epsilon 1,12(s,6H) ; 1,22 (s,6H) ; 1,28(t,3H) ; 2,99(q,2H).
Spectre IR :
C=N : 1640 cm⁻¹

### Exemple 5 : 3-phényl 5,5,6,6-tétraméthyl 7-éthylthio 1-oxa 2,4-diazabicyclo [3.2.0.] hept-2-ène.

F = 132-134°C. Rendement 62%

### Exemple 6 : 3-(O-nitrophényl) 5,5,6,6-tétraméthyl 7-éthylthio 1-oxa 2,4-diazabicyclo [3.2.0.] hept-2-ène.

F = 90-91°C. Rendement 42%.

### Exemple 7 : 7-éthoxy 1-(p-nitrophényl) 3-phényl 5,5,6,6-tétraméthyl 1,2,4-triazabicyclo [3.2.0.] hept-2-ène.

On ajoute de la triéthylamine (4 équivalents) dans une solution de 3,3,4,4-tétraméthyl 2-éthoxy 1-azétine (1,46 mmol) et d'alpha-chlorobenzaldéhyde (p-nitrophényl) hydrazone (1,75 mmol) dans 20 cm₃ de benzène, sous atmosphère inerte. On chauffe au reflux jusqu'à complète réaction, filtre le chlorhydrate de triéthylamine formé, élimine le solvant sous pression réduite, purifie le résidu par tituration dans l'éther de pétrole (eb. 60-80°) et obtient le produit attendu (rendement 89%). F = 141-143°C après recristallisation dans l'éther de pétrole (Eb. 40-60°C).

### Préparation de l'alpha-chlorobenzaldéhyde (p-nitrophényl) hydrazone.

### a) N-benzoyl N′-(p-nitrophényl) hydrazide.

On ajoute 2 g de chlorure de benzoyle (0,014 mmol) dans une solution de p-nitrophényl hydrazine (0,014 mmol) dans 10 cm₃ de pyridine. An agite 20 minutes, verse dans l'eau glacée, agite, filtre le précipité, le lave à l'eau puis le sèche à 100°C. Après recristallisation, on obtient le produit attendu fondant à 190-192°C (rendement 83%).

### b) alpha-chlorobenzaldéhyde (p-nitrophényl) hydrazone.

On dissout dans 50 cm₃ d'éther le produit obtenu au stade a) (0,07 mmol) et du pentachorure de phosphore (0,07 mmol) puis chauffe 16 heures au reflux. On refroidit, ajoute 30 g de phénol en solution dans 50 cm₃ d'éther puis 40 cm₃ de méthanol. On élimine partiellement le solvant, refroidit et maintient plusieurs jours à +4°C. On obtient le produit attendu cristallisé. F = 188-190°C (rendement 79%).

### Exemple 8 : 7-éthylthio 1-(p-nitrophényl) 3-phényl 5,5,6,6-tétraméthyl 1,2,4-triazabicyclo [3.2.0.] hept-2-ène.

En opérant comme indiqué à l'exemple 7 en utilisant au départ la 2-éthylthio 1-azétine correspondant, on a obtenu le produit attendu. F = 153-154°C (rendement 88%).

### Exemple 9 : 7-éthoxy 3-phényl 1-(p-nitrophényl) 5,5,6,6-tétraméthyl 2,4-diazabicyclo [3.2.0.] hept-2-ène.

On ajoute sous atmosphère inerte de la triéthylamine (1,5 équivalents) dans une solution de 3,3,4,4-tétraméthyl 2-éthoxy azétine (2,18 mmol) et de chlorure d'imidoyle (2,18 mmol) dans 20 cm₃ de benzène. On agite à température ambiante pendant plusieurs heures. On élimine par filtration le précipité formé, concentre à sec sous pression réduite, chromatographie le résidu sur silice et obtient le produit attendu. F = 165-167°C après recristallisation dans l'éther de pétrole (Eb. 60-80°C) (rendement 46%).

### Préparation du N-(p-nitrobenzyl) benzimidoyl chloride.

On chauffe au reflux pendant 30 minutes sous atmosphère inerte 3,7 mmol de N-(-p-nitrobenzyl) benzamide en solution dans du chlorure de thionyle. On refroidit, élimine l'excès de solvant sous atmosphère inerte, cristallise le résidu dans le cyclohexane sous azote et obtient le produit attendu, utilisé tel quel dans la suite de la synthèse.

### Exemple 10 : 7-éthylthio 3-phényl 1-(p-nitrophényl) 5,5,6,6-tétraméthyl 2,4-diazabicyclo [3.2.0.] hept-2-ène.

En opérant comme indiqué à l'exemple 9 en utilisant au départ la 2-éthylthio 1-azétine correspondante, on a obtenu le produit attendu. F = 148-149°C (rendement 68%).

### Exemple 11 : 7a-éthoxy 3-(4-méthoxyphényl) 5,6,7,7a-tétrahydropyrrolo [1,2-d] [1,2,4] oxadiazol-5-one.

On mélange 2,27 g de O-éthylsuccinimide (K. Matoba and T. Yamazaki, Chem., Pharm. Bull, 22 (12), 2999-3001 (1974) et 1,86 g de chlorure de 4-méthoxybenzohydroximinoyl dans 50 cm₃ de benzène, à température ambiante sous atmosphère inerte puis ajoute 1,31 g de triéthylamine. On maintient sous agitation pendant 4 jours à température ambiante et filtre le précipité de chlorure de triéthylammonium formé. On concentre à sec le filtrat sous pression réduite, purifie le résidu par chromatographie sur silice (éluant : chlorure de méthylène) et obtient 0,49 g de produit attendu. F = 114-116°C après recristallisation dans le mélange éther-éther de pétrole (Eb. 40-60°C).
Spectre IR (CHCl₃ solution) :
2980, 1750, 1610, 1365, 1250, 1100, 870 et 835 cm⁻¹.
Spectre RMN (CDCl₃) :
Epsilon 1,23(3H,t,J=7,2 Hz, -CH₃) ; 2,50-2,77(3H,m,C₆-H, 2xC₇-H) ; 3,00-3,06(1H,q de AB doublet, J_{H6H6′}, 16,9Hz, J_{H6,H7} 11,6Hz, H_{H6,H7′} 8,05Hz, C₆-H) ;3,35-3,61 (2H,q de AB quartet, J_{AB} 8,9Hz, J_{AX}=J_{BX} 7,2Hz, -OCH₂) ;3,85 (3H,s,-OCH₃) ; 6,95 et 7,79 (4H, ABq, aromatique).

### Exemple 12 :

On a préparé des comprimés répondant à la formulation suivante :

| | |
|---|---|
| - Produit de l'exemple 1 | 100 mg |
| - Excipient q.s. pour un comprimé terminé à | 200 mg |

(Détail de l'excipient : lactose, amidon, talc, stéarate de magnésium).

### Exemple 13 :

On a préparé des comprimés répondant à la formulation suivante :

| | |
|---|---|
| - Produit de l'exemple 4 | 100 mg |
| - Excipient q.s. pour un comprimé terminé à | 200 mg |

(Détail de l'excipient : lactose, amidon, talc, stéarate de magnésium).

### Exemple 14 :

On a préparé un soluté injectable répondant à la formulation suivante :

| | |
|---|---|
| - Produit de l'exemple 1 | 500 mg |
| - Excipient aqueux stérile | 5 cm₃ |

### Exemple 15 :

On a préparé des gélules répondant à la formulation suivante :

| | |
|---|---|
| - Produit de l'exemple 5 | 250 mg |
| - Excipient q.s. pour un gélule terminée à | 400 mg |

(Détail de l'excipient : talc, stéarate de magnésium, aérosil).

### ETUDE PHARMACOLOGIQUE

Comme on l'a mentionné précédemment, les composés selon l'invention présentent des propriétés pharmacologiques intéressantes. Ils ont été soumis, notamment, à l'épreuve de l'activité comme inhibiteur de la synthèse des éicosanoïdes parles neutrophiles péritonéaux du cobaye, après adjonction d'acide arachidonique ¹⁴C et d'une substance porteuse d'ions calcium, au moyen d'une modofication de la méthode publiée par J. Harvey et D.J. Osborne dans J. Pharmacol. Methods, 9, (2), 147-155 (1983). Les composés selon l'invention inhibent de manière sélective la synthèse du produit de la cyclo oxygénase (TX B₂ et 5-HHT). Ils exercent des effets moins marqués sur la synthèse du produit dela 5-lypoxygénase (5-HETE).

Dans ces tests, les composés de l'exemple 4 présente une CI₅₀ (micromolaire) de5,0 (TX B 2), 6,3 (5-HHT) et > 100 (5-HETE).

## Revendications

1. Nouveaux dérivés d'azabicycloheptène de formule générale (I) : 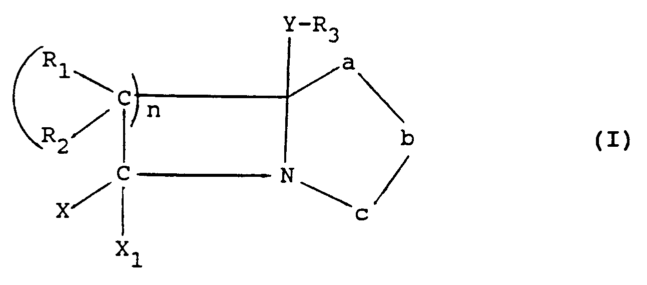 dans laquelle :
- R₁, R₂ identiques ou différents, représentent un atome d'hydrogène, un radical alkyle renfermant de 1 à 5 atomes de carbone,
- n représente un nombre entier égal à 1 ou 2,
- X et X₁ représentent chacun un atome d'hydrogène ou un radical alkyle renfermant de 1 à 3 atomes de carbone, ou X et X₁ forment ensemble avec l'atome de carbone auxquels ils sont liés un groupement O=C-,
- Y représente un atome d'oxygène ou un atome de soufre,
- R₃ représente un radical alkyle renfermant de 1 à 3 atomes de carbone ou un groupement phényle, 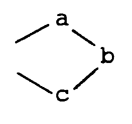 représente des groupements 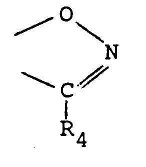 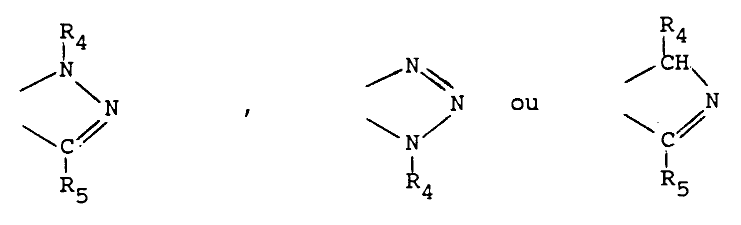 dans lesquels R₄ et R₅ identiques ou différents représentent un groupement phényle ou un groupement phényle substitué par un atome d'halogène, un radical alkyle renfermant de 1 à 3 atomes de carbone, un radical alcoxy renfermant de 1 à 3 atomes de carbone, un groupement nitro, ainsi que leurs sels.

2. Dérivés répondant à la formule (I) de la revendication 1, dans laquelle :
- R₁, R₂, identiques ou différents, représentent un atome d'hydrogène, un radical méthyle,
- X et X₁, identiques ou différents, représentent un atome d'hydrogène ou un radical méthyle, ou X et X₁ forment ensemble avec l'atome de carbone auxquels ils sont liés un groupement 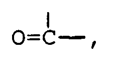
- R₃ représente un radical méthyle ou éthyle, n, Y et 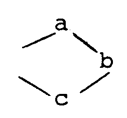 ont la signification déjà indiquée, ainsi que leurs sels.

3. Dérivés répondant à la formule (I) de la revendication 1 ou 2, dans laquelle :
- R₁, R₂, X, X₁ représentent un radical méthyle,
- R₃ représente un radical éthyle,
- R₄ et R₅, identiques ou différents, représentent un groupement phényle ou un groupement phényle substitué par un radical méthoxy ou par un groupement nitro,
- n et Y ont la signification déjà indiquée, ainsi que leurs sels.

4. Dérivés répondant à la formule (I) de la revendication 1, 2 ou 3, dans laquelle n est égal à 1, ainsi que leurs sels.

5. Dérivés répondant à la formule (I) de la revendication 1, 2 ou 3, dans laquelle n est égal à 2, ainsi que leurs sels.

6. Le 3-(p-méthoxyphényl) 5,5,6,6-tétraméthyl 7-éthoxy 1-oxa 2,4-diazabicyclo [3.2.0.] hept-2-ène.

7. Le 3-phényl 5,5,6,6-tétraméthyl 7-éthoxy 1-oxa 2,4-diazabicyclo [3.2.0.] hept-2-ène.

8. Le 3-(O-nitrophényl) 5,5,6,6-tétraméthyl 7-éthoxy 1-oxa 2,4-diazabicyclo [3.2.0.] hept-2-ène.

9. Le 3-(p-méthoxyphényl) 5,5,6,6-tétraméthyl 7-éthylthio 1-oxa 2,4-diazabicyclo [3.2.0.] hept-2-ène.

10. Le 3-phényl 5,5,6,6-tétraméthyl 7-éthylthio 1-oxa 2,4-diazabicyclo [3.2.0.] hept-2-ène.

11. Le 3-(O-nitrophényl) 5,5,6,6-tétraméthyl 7-éthylthio 1-oxa 2,4-diazabicyclo [3.2.0.] hept-2-ène.

12. Le 7-éthoxy 1-(p-nitrophényl) 3-phényl 5,5,6,6-tétraméthyl 1,2,4-triazabicyclo [3.2.0.] hept-2-ène.

13. Le 7-éthylthio 1-(p-nitrophényl) 3-phényl 5,5,6,6-tétraméthyl 1,2,4-triazabicyclo [3.2.0.] hept-2-ène.

14. Le 7-éthoxy 3-phényl 1-(p-nitrophényl) 5,5,6,6-tétraméthyl 2,4-diazabicyclo [3.2.0.] hept-2-ène.

15. Le 7-éthylthio 3-phényl 1-(p-nitrophényl) 5,5,6,6-tétraméthyl 2,4-diazabicyclo [3.2.0.] hept-2-ène.

16. Procédé de préparation des nouveaux dérivés d'azabicycloheptène tels que définis par la formule (I) de la revendication 1, ainsi que de leurs sels, caractérisé en ce que l'on effectue une cycloaddition entre un produit de formule (II) : 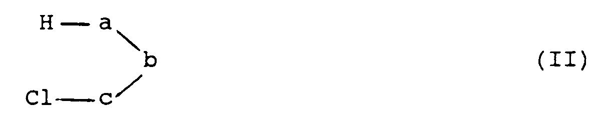 xdans laquelle le groupement 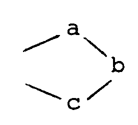 a la signification déjà indiquée et un produit de formule (III) : 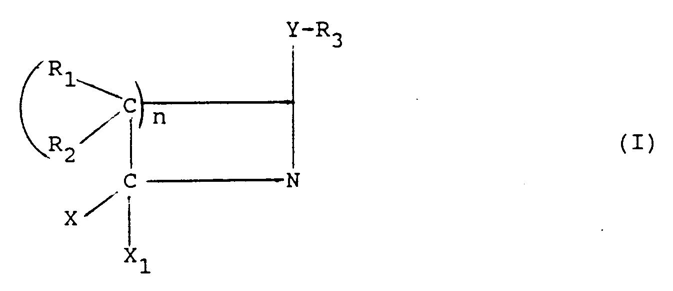 dans laquelle R₁, R₂, n, X, X₁, Y et R₃ ont la signification déjà indiquée, pour obtenir le produit de formule (I) dans laquelle R₁, R₂, n, X, X₁, Y, R₃, 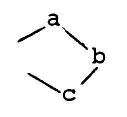 ont la signification déjà indiquée que l'on peut salifier le cas échéant.

17. Procédé de préparation selon la revendication 16, caractérisé en ce que la cycloaddition entre le produit de formule (II) et le produit de formule (III) est effectuée au sein d'un solvant organique apolaire tel que le benzène en présence d'une amine telle que la triéthylamine.

18. Médicaments, caractérisé en ce qu'ils sont constitués par les nouveaux dérivés d'azabicycloheptène tels que définis par la formule (I) de la revendication 1, ainsi que par leurs sels pharmaceutiquement acceptables.

19. Médicaments, caractérisé en ce qu'ils sont constitués par les nouveaux dérivés d'azabicycloheptène tels que définis à l'une quelconque des revendications 2 à 15, ainsi que par leurs sels pharmaceutiquement acceptables.

20. Compositions pharmaceutiques, caractérisées en ce qu'elles renferment à titre de principe actif, l'un au moins des médicaments tels que définis à l'une quelconque des revendications 18 ou 19.

## Claims

1. New azabicycloheptene derivatives of general formula (I): 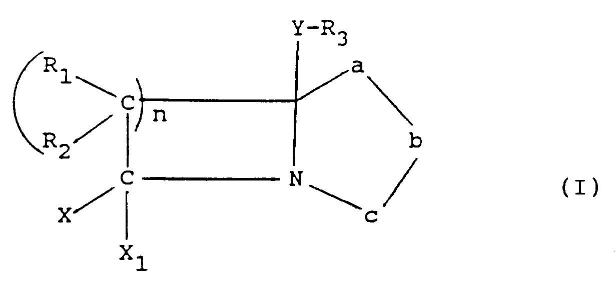 in which:
- R₁, R₂, identical or different, represent a hydrogen atom, an alkyl radical containing 1 to 5 carbon atoms,
- n represents an integer equal to 1 or 2,
- X and X₁ each represent a hydrogen atom or an alkyl radical containing 1 to 3 carbon atoms, or X and X₁ form together with the carbon atom to which they are linked an O=C- group,
- Y represents an oxygen atom or a sulphur atom,
- R₃ represents an alkyl radical containing 1 to 3 carbon atoms or a phenyl group, 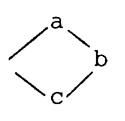 represents 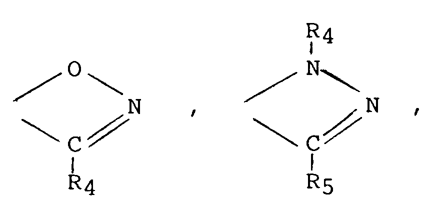 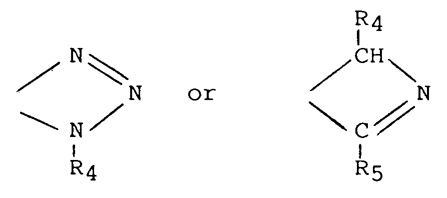 groups
in which R₄ and R₅, identical or different, represent a phenyl group or a phenyl group substituted by a halogen atom, an alkyl radical containing 1 to 3 carbon atoms, an alkoxy radical containing 1 to 3 carbon atoms, a nitro group, as well as their salts.

2. Derivatives corresponding to formula (I) of claim 1, in which:
- R₁, R₂, identical or different, represent a hydrogen atom, a methyl radical,
- X and X₁, identical or different, represent a hydrogen atom or a methyl radical, or X and X₁ form together with the carbon atom to which they are linked an 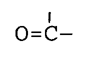 group,
- R₃ represents a methyl or ethyl radical, n, Y and 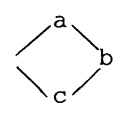 have the meaning already indicated, as well as their salts.

3. Derivatives corresponding to formula (I) of claim 1 or 2, in which:
- R₁, R₂, X, X₁ represent a methyl radical,
- R₃ represents an ethyl radical,
- R₄ and R₅, identical or different, represent a phenyl group or a phenyl group substituted by a methoxy radical or by a nitro group,
- n and Y have the meaning already indicated, as well as their salts.

4. Derivatives corresponding to formula (I) of claim 1, 2 or 3, in which n is equal to 1, as well as their salts.

5. Derivatives corresponding to formula (I) of claim 1, 2 or 3, in which n is equal 2, as well as their salts.

6. 3-(p-methoxyphenyl) 5,5,6,6-tetramethyl 7-ethoxy 1-oxa 2,4-diazabicyclo [3.2.0.] hept-2-ene.

7. 3-phenyl 5,5,6,6-tetramethyl 7-ethoxy 1-oxa 2,4-diazabicyclo [3.2.0.] hept-2-ene.

8. 3-(O-nitrophenyl) 5,5,6,6-tetramethyl 7-ethoxy 1-oxa 2,4-diazabicyclo [3.2.0.] hept-2-ene.

9. 3-(p-methoxyphenyl) 5,5,6,6-tetramethyl 7-ethylthio 1-oxa 2,4-diazabicyclo [3.2.0.] hept-2-ene.

10. 3-phenyl 5,5,6,6-tetramethyl 7-ethylthio 1-oxa 2,4-diazabicyclo [3.2.0.] hept-2-ene.

11. 3-(O-nitrophenyl) 5,5,6,6-tetramethyl 7-ethylthio 1-oxa 2,4-diazabicyclo [3.2.0.] hept-2-ene.

12. 7-ethoxy 1-(p-nitrophenyl) 3-phenyl 5,5,6,6-tetramethyl 1,2,4-triazabicyclo [3.2.0.] hept-2-ene.

13. 7-ethylthio 1-(p-nitrophenyl) 3-phenyl 5,5,6,6-tetramethyl 1,2,4-triazabicyclo [3.2.0.] hept-2-ene.

14. 7-ethoxy 3-phenyl 1-(p-nitrophenyl) 5,5,6,6-tetramethyl 2,4-diazabicyclo [3.2.0.] hept-2-ene.

15. 7-ethylthio 3-phenyl 1-(p-nitrophenyl) 5,5,6,6-tetramethyl 2,4-diazabicyclo [3.2.0.] hept-2-ene.

16. Preparation process for new azabicycloheptene derivatives as defined by formula (I) of claim 1, as well as for their salts, characterized in that a cycloaddition is carried out between a product of formula (II): 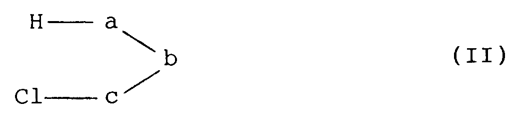 in which the group 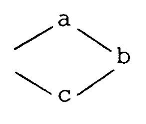 has the meaning already indicated, and a product of formula (III): 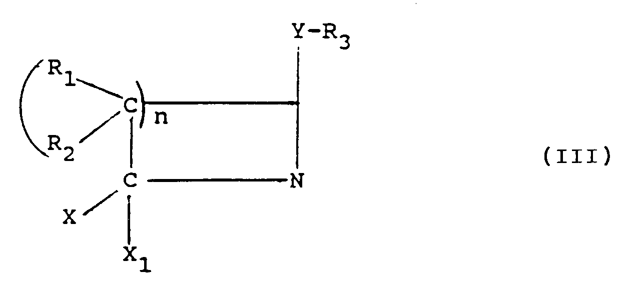 in which R₁, R₂, n, X, X₁, Y and R₃ have the meaning already indicated, in order to obtain the product of formula (I) in which R₁, R₂, n, X, X₁, Y, R₃, 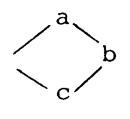 have the meaning already indicated, which if appropriate can be salified.

17. Preparation process according to claim 16, characterized in that the cycloaddition between the product of formula (II) and the product of formula (III) is carried out in an apolar organic solvent such as benzene in the presence of an amine such as triethylamine.

18. Medicaments, characterized in that they are constituted by the new azabicycloheptene derivatives as defined by formula (I) of claim 1, as well as by their pharmaceutically acceptable salts.

19. Medicaments, characterized in that they are constituted by the new azabicycloheptene derivatives as defined in any one of claims 2 to 15, as well as by their pharmaceutically acceptable salts.

20. Pharmaceutical compositions, characterized in that they contain as active ingredient, at least one of the medicaments as defined in any one of claims 18 or 19.

## Patentansprüche

1. Neuartige Azabicycloheptenderivate mit der allgemeinen Formel (I): 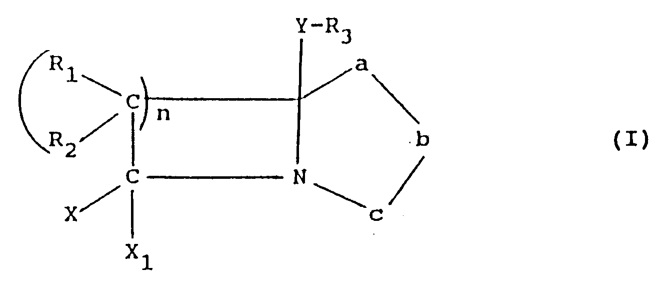 in der:
- R₁, R₂ , gleich oder verschieden, für ein Wasserstoffatom, für einen Alkylrest mit 1 bis 5 Kohlenstoffatomen stehen,
- n für eine ganze Zahl steht, die gleich 1 oder 2 ist,
- X und X₁ jeweils für ein Wasserstoffatom stehen oder für einen Alkylrest mit 1 bis 3 Kohlenstoffatomen oder X und X₁ zusammen mit dem Kohlenstoffatom, an das sie gebunden sind, eine (O=C-)-Gruppierung bilden,
- Y für ein Sauerstoffatom oder für ein Schwefelatom steht,
- R₃ für einen Alkylrest mit 1 bis 3 Kohlenstoffatomen oder eine Phenylgruppierung steht, 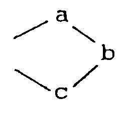 für folgende Gruppierungen steht: 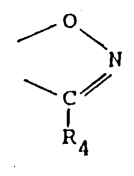 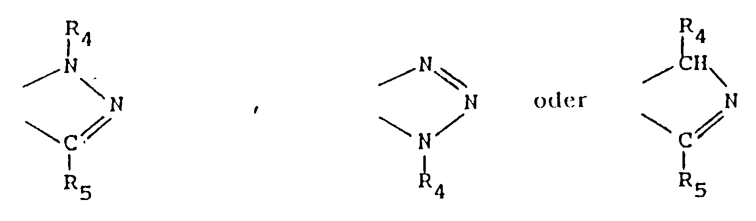 in denen R₄ und R₅, gleich oder verschieden, für eine Phenylgruppierung steht oder für eine Phenylgruppierung, die mit einem Halogenatom, einem Alkylrest mit 1 bis 3 Kohlenstoffatomen, einem Alkoxyrest mit 1 bis 3 Kohlenstoffatomen, einer Nitrogruppierung substituiert ist, sowie ihre Salze.

2. Derivate, die der Formel (I) von Anspruch 1 entsprechen, in der:
- R₁, R₂, gleich oder verschieden, für ein Wasserstoffatom, für einen Methylrest stehen,
- X und X₁, gleich oder verschieden, für ein Wasserstoffatom oder für einen Methylrest stehen oder X und X₁ zusammen mit dem Kohlenstoffatom, an das sie gebunden sind, eine 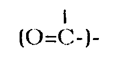 Gruppierung bilden,
- R₃ für einen Methyl- oder Ethylrest steht, n, Y und 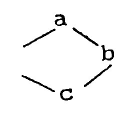 die schon angegebene Bedeutung haben, sowie ihre Salze.

3. Derivate, die der Formel (I) von Anspruch 1 oder 2 entsprechen, in der:
- R₁, R₂, X, X₁ für einen Methylrest stehen,
- R₃ für einen Ethylrest steht,
- R₄ und R₅, gleich oder verschieden, für eine Phenylgruppierung steht oder für eine Phenylgruppierung, die mit einem Methoxyrest oder einer Nitrogruppierung substituiert ist,
- n und Y die schon angegebene Bedeutung haben, sowie ihre Salze.

4. Derivate, die der Formel (I) von Anspruch 1, 2 oder 3 entsprechen, in der n gleich 1 ist, sowie ihre Salze.

5. Derivate, die der Formel (I) von Anspruch 1, 2 oder 3 entsprechen, in der n gleich 2 ist, sowie ihre Salze.

6. Das 3-(p-Methoxyphenyl)-5,5,6,6-tetramethyl-7-ethoxy-1-oxa-2,4-diaza-bicyclo[3.2.0]hept-2-en.

7. Das 3-Phenyl-5,5,6,6-tetramethyl-7-ethoxy-1-oxa-2,4-diaza-bicyclo[3.2.0]hept-2-en.

8. Das 3-(O-Nitrophenyl)-5,5,6,6-tetramethyl-7-ethoxy-1-oxa-2,4-diaza-bicyclo[3.2.0]hept-2-en.

9. Das 3-(p-Methoxyphenyl)-5,5,6,6-tetramethyl-7-ethylthio-1-oxa-2,4-diaza-bicyclo[3.2.0]hept-2-en.

10. Das 3-Phenyl-5,5,6,6-tetramethyl-7-ethylthio-1-oxa-2,4-diaza-bicyclo[3.2.0]hept-2-en.

11. Das 3-(O-Nitrophenyl)-5,5,6,6-tetramethyl-7-ethylthio-1-oxa-2,4-diaza-bicyclo[3.2.0]hept-2-en.

12. Das 7-Ethoxy-1-(p-nitrophenyl)-3-phenyl-5,5,6,6-tetramethyl-1,2,4-triaza-bicyclo[3.2.0]hept-2-en.

13. Das 7-Ethylthio-1-(p-nitrophenyl)-3-phenyl-5,5,6,6-tetramethyl-1,2,4-triaza-bicyclo[3.2.0]hept-2-en.

14. Das 7-Ethoxy-3-phenyl-1-(p-nitrophenyl)-5,5,6,6-tetramethyl-2,4-diaza-bicyclo[3.2.0]hept-2-en.

15. Das 7-Ethylthio-3-phenyl-1-(p-nitrophenyl)-5,5,6,6-tetramethyl-2,4-diaza-bicyclo[3.2.0]hept-2-en.

16. Verfahren zur Herstellung der neuartigen Azabicycloheptenderivate, wie sie in Formel (I) von Anspruch 1 definiert sind, sowie ihrer Salze, dadurch gekennzeichnet, daß man eine Cycloaddition durchführt eines Produkts mit der Formel (II): 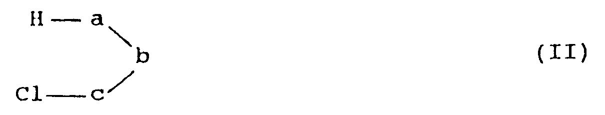 in der die Gruppierung 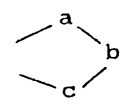 die schon allgegebene Bedeutung hat, an ein Produkt mit der Formel (III): 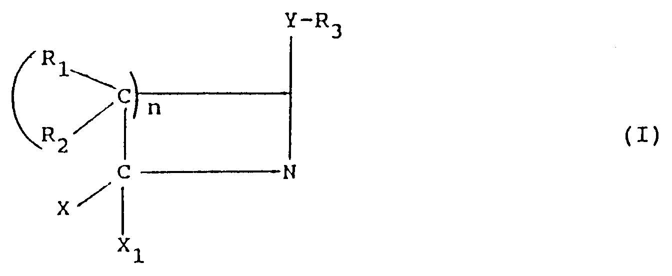 in der R₁, R₂, n, X, X₁, Y und R₃ die schon angegebene Bedeutung haben, wodurch man ein Produkt mit der Formel (I) erhält, in der R₁, R₂, n, X, X₁, Y, R₃, 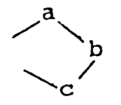 die schon angegebene Bedeutung haben, aus dem man gegebenenfalls ein Salz bilden kann.

17. Herstellungsverfahren gemäß Anspruch 16, dadurch gekennzeichnet, daß man die Cycloaddition des Produkts mit der Formel (II) an das Produkt mit der Formel (III) in einem apolaren organischen Lösungsmittel wie Benzol in Gegenwart eines Amins wie Triethylamin durchführt.

18. Arzneistoffe, dadurch gekennzeichnet, daß sie aus den neuartigen Azabicycloheptenderivaten bestehen, wie sie in Formel (I) definiert sind, sowie aus ihren pharmezeutisch verwendbaren Salzen.

19. Arzneistoffe, dadurch gekennzeichnet, daß sie aus den neuartigen Azabicycloheptenderivaten bestehen, wie sie in einem beliebigen der Ansprüche 2 bis 15 definiert sind, sowie aus ihren pharmazeutisch verwendbaren Salzen.

20. Pharmazeutische Zusammensetzungen, dadurch gekennzeichnet, daß sie als Wirkstoff wenigstens einen der Arzneistoffe enthalten, wie sie in irgendeinem der Ansprüche 18 oder 19 definiert sind.
